# EUROPEAN PATENT APPLICATION

(11) **EP 2 641 585 A1**
(43) Date of publication of application: **25.09.2013**
(21) Application number: 12160214.8
(22) Date of filing: 19.03.2012
(51) Int. Cl.: A61K 8/34, A61K 8/89, A61K 8/92, A61K 8/97, A61Q 19/00, A61P 17/00, A61K 36/738

(54) **Cosmetic skin composition with soothing effect and its use**

(71) Applicant: Coty Germany GmbH, 55116 Mainz (DE)
(72) Inventor: Doucet, Olivier, 06190 Roquebrune Cap Martin (FR); Pujos, Muriel, F-06200 Nice (FR); Robert, Cécile, F-06300 Nice (FR); Desriaux, Elisabeth, F-06500 Menton (FR); Bernini, Dorothee, MC-98000 Monaco (MC)
(74) Representative: Gulde Hengelhaupt Ziebig & Schneider

(57) **Abstract**

The present invention relates to a skin composition with excellent soothing activity and its use for soothing the skin. The composition comprises a lipophilic complex containing a Rosa centifolia flower extract with significant soothing activity.

## Description

The present invention relates to a skin composition with excellent soothing activity and its use for soothing the skin by applying it on skin areas irritated by environmental conditions. The composition comprises a lipophilic complex containing a Rosa centifolia flower extract with significant soothing activity.

According to the invention irritated skin means skin which is irritated by environmental conditions, e.g. pollution, UV radiation or air conditioning in closed rooms. The features of irritated skin which is caused by these environmental conditions may be redness, itching, tightening and/or swelling. Sometimes irritated skin shows light inflammatory reactions.

It is well known in cosmetics to apply to irritated skin vegetal extracts like centella asiatica, avena sativa (oat) extract or chamomile extract, but their use may induce skin allergy reactions. For instance, the use of chamomile-containing preparations is contraindicated in persons with hypersensitivity to ragweed pollens. Due to its content in natural bisabolol, it may induce contact dermatitis, whereas synthetic bisabolol does not. Several cases of oat sensitization have been reported in children with atopic dermatitis with cosmetics containing oat proteins and it is suggested that they should avoid the use of such products. Centella asiatica is another sensitizer which can induce allergic contact dermatitis.

It was the problem of the present invention to provide a composition with superior soothing effect on skin which does not have the disadvantages of the prior art.

It was found by the inventors of the present application that this problem can be solved with a cosmetic composition which comprises 0,1 to 3,0 wt%, preferably 0,3 to 2,0 wt%, and especially preferred 1,8 to 2,0 wt of a lipophilic complex containing 0,1 to 1,0 wt% of Rosa centifolia flower extract obtainable by cryoextraction of Rosa centifolia flowers, 0,1 to 1,0 wt% of Bisabolol (and) Farnesol, 0,01 to 0,1 wt% of antioxidants, 5,0 to 6,0 wt% of Calendula Oil, 2,0 to 4,0 wt% Camelina Sativa Seed Oil and cosmetically acceptable auxiliary substances up to 100 wt%, whereby these auxiliary substances comprise 55 to 74 wt% of a silicone elastomer, wherein all given percentages relate to the total weight of the composition.

The lipophilic complex which is used in the composition according to the invention is composed of 0,1 to 1,0 wt% of Rosa centifolia flower extract and 99,0 to 99,9 wt% of Caprylic/Capric triglyceride. This complex provides a significant soothing effect. It is assumed that this significant soothing effect is caused by the Rosa centifolia flower extract which was obtained by cryoextraction of Rosa centifolia petals coming preferably from Grasse roses. The first step of the extraction method consists in freezing the fresh flowers, preferably in the place of harvest, at -10 to -40°C, preferably at -28°C. Then the flowers are subjected to cryopulverization under nitrogen at about ― 196°C and subsequent extraction by maceration of the obtained cryopulverized powder in Caprylic/Capric Triglyceride. The composition of the invention comprises a small amount of 0,1 to 1,0 wt% of an anti-irritant which is Bisabolol (and) Farnesol (Trade name: Dragosantol). Preferably it is contained from 0,3 to 0,8 wt% and most preferred from 0,4 to 0,6 wt%.

According to the invention the composition further comprises 0,01 to 0,1 wt% of known and in cosmetics widely used antioxidants, preferably 0,03 to 0,08 wt% and most preferred 0,04 to 0,06 wt%. An especially preferred antioxidant used according to the invention is an antioxidant blend comprising PEG-8, tocopherol, ascorbyl palmitate, citric acid and ascorbic acid (Trade name: Oxynex K).

The composition of the invention additionally comprises 5,0 to 6,0 wt% of Calendula Oil (Pale, NA 20385; Caprylic/Capric Triglyceride & Glycine Soja (Soybean) Oil & Calendula Officinalis Flower Extract), preferably 5,0 to 5,8 wt%, and 2,0 to 4,0 wt% of Camelina Sativa Seed Oil (Huile Cameline Raffinee C05002), preferably 2,5 to 3,5 wt%.

The composition of the invention is formulated as serum oil concentrate and contains 55 to 74 wt%, preferably 68 to 74 wt%, of a silicone elastomer. Suitable silicone elastomers which can be used according to the invention are for instance Dimethicone (and) Dimethicone Crosspolymer (Dow Corning^{®} 9041) or Cyclopentasiloxane (and) Dimethicone Crosspolymer (Dow Corning^{®} 9040). Preferably Cyclopentasiloxane (and) Dimethicone Crosspolymer (Dow Corning^{®} 9040) is used.

The composition of the invention comprises further cosmetically acceptable auxiliary substances which are selected from the group consisting of emollients, skin conditioning agents, viscosity regulating substances, fragrances, and mixtures thereof.

According to the invention emollients and skin conditioning agents can be added to soften and smoothen the skin and to improve the sensory effects on skin. Suitable emollients are for instance Isononyl Isononanoate (Trade name: DUB ININ) and Pentaerythrityl Tetraethylhexanoate (Trade name: DUB PTO). Suitable skin conditioners are for instance Cyclopentasiloxane (and) Dimethiconol (Trade name: Dow Corning 1501 Fluid) and Pentaerythrityl Tetraethylhexanoate (Trade name: DUB PTO).

In a preferred embodiment of the invention the composition comprises fragrances in a range of 2,0 to 4,0 wt% to improve the odor of the cosmetic product. Preferably natural fragrances, such as plant or fruit extracts are used.

The composition of the present invention has excellent soothing activity on skin irritated by environmental conditions. Therefore, it is another object of the present application to provide a composition for use in soothing the skin by applying it on skin areas irritated by environmental conditions, wherein the composition comprises 0,1 to 3,0 wt%, preferably 0,3 to 2,0 wt%, of a lipophilic complex containing 0,1 to 1,0 wt% of Rosa centifolia flower extract obtainable by cryoextraction of Rosa centifolia flowers, 0,1 to 1,0 wt% of Bisabolol (and) Farnesol, 0,01 to 0,1 wt% of antioxidants, 5,0 to 6,0 wt% of Calendula Oil, 2,0 to 4,0 wt% Camelina Sativa Seed Oil and cosmetically acceptable auxiliary substances up to 100 wt%, whereby these auxiliary substances comprise 55 to 74 wt% of a silicone elastomer, wherein all given percentages relate to the total weight of the composition.

The composition of the present invention does not only provide a superior soothing effect, it also provides a hydrating and antioxidative effect. It was found that the composition helps to recover an ideal balance to the face, provides a healthy look and exceptional radiance. The skin is fresh, smooth, velvety and replenished again.

The compositions of the present invention are prepared in a manner as well known in cosmetic industries. Details of the preparation are given in the examples.

The following examples are offered to illustrate the cosmetic composition of the present invention. They are not intended to be limiting in any respect.

### Example 1: Serum oil concentrate

| | | Batch 1 | Batch 2 | Batch 3 |
|---|---|---|---|---|
| | | % | % | % |
| **PHASE A** | CYCLOPENTASILOXANE & DIMETHICONOL | 8,00 | 15,00 | 20,00 |
| | PENTAERYTHRlTYL TETRAETHYLHEXANOATE | 6,00 | 4,50 | 2,00 |
| | ISONONYL ISONONANOATE | 2,00 | 3,50 | 6,00 |
| | | | | |
| **PHASE B** | CYCLOPENTASILOXANE & DIMETHICONE CROSSPOLYMER | 73,37 | 64,14 | 57,12 |
| | | | | |
| **PHASE C** | CALENDULA OIL | 5,00 | 5,50 | 6,00 |
| | CAMELINA SATIVA SEED OIL | 2,00 | 2,80 | 3,50 |
| | BISABOLOL & FARNESOL | 0,30 | 0,60 | 0,80 |
| | ROSE COMPLEX (GRASSE) LIPO | 1,80 | 1,90 | 2,00 |
| | PEG-8 & TOCOPHEROL & ASCORBYL PALMITATE & ASCORBIC ACID | 0,03 | 0,06 | 0,08 |
| | | | | |
| **PHASE D** | FRAGRANCE | 2,00 | 2,50 | 3,50 |

The composition is prepared as follows:
The ingredients of phase A are added in the main vessel under stirring to homogeneity. Phase B is slowly added to phase A by slowly stirring. It is preferably used a paddle stirrer. The stirring rate is preferably from 50 to 150 rpm. Phase C is added to the main phase under the same conditions by stirring until homogeneity. Finally all ingredient of phase D is added and stirred until homogeneity is achieved.

### Example 2: Determination of skin hydration degree

Skin hydration of the epidermal outermost layers of the arms of 12 women was assessed before application and after application of the composition of Example 1 during 2 hours using the Corneometer™ (Courage & Khazaka Electronics). This technique is widely used to investigate skin water content and to quantify the effect of cosmetics or dermatological products. Due to the high dielectric constant of water, the electrical characteristics of the *stratum corneum* depend on its water content. The values of electrical capacitance are given in arbitrary units (A.U.) settled by the instrument on a scale from 0 A.U. to about 130 A.U. These values show evidence of the hydration degree of the outermost layers of the epidermis at a given time.

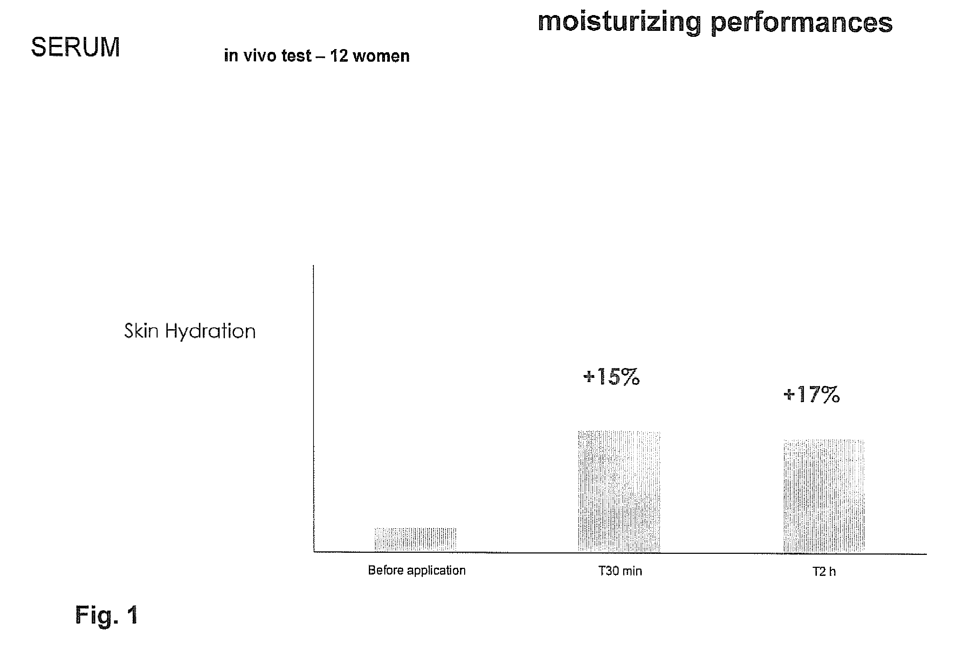

It was found that 30 minutes after application of the composition of Example 1 the hydration degree of the treated skin areas increased for 15% and after 2 hours it was 17% higher in comparison to the skin hydration degree on non treated areas at the same times of measurements. The results are demonstrated in Figure 1.

### Example 3: Determination of radical protecting activity

The Radical Protection Factor (RPF) which determines the activity in respect of the bonding of free radicals by an antioxidant was measured as described in WO 99/66881. The RPF of the serum oil of Example 1 was found to be 30 · 10¹⁴ radicals/mg.

## Claims

1. A skin composition with soothing effect comprising
0,1 to 3,0 wt% of a lipophilic complex containing 0,1 to 1,0 wt% of Rosa centifolia flower extract obtainable by cryoextraction of Rosa centifolia flowers, 0,1 to 1,0 wt% of Bisabolol (and) Farnesol, 0,01 to 0,1 wt% of antioxidants, 5,0 to 6,0 wt% of Calendula Oil, 2,0 to 4,0 wt% Camelina Sativa Seed Oil, and cosmetically acceptable auxiliary substances up to 100 wt%, whereby these auxiliary substances comprise 55 to 74 wt% of a silicone elastomer,
wherein all given percentages relate to the total weight of the composition.

2. The composition according to claim 1,
wherein the composition comprises 0,3 to 2,0 wt% of the lipophilic complex, preferably 1,8 to 2,0 wt%.

3. The composition according to claim 1 or 2,
wherein the antioxidant is an antioxidant blend comprising PEG-8, tocopherol, ascorbyl palmitate, citric acid and ascorbic acid.

4. The composition according to any one of claims 1 to 3,
wherein the composition comprises 0,03 to 0,08 wt% of antioxidants, preferably 0,04 to 0,06 wt%.

5. The composition according to any one of claims 1 to 4,
wherein the composition comprises 0,3 to 0,8 wt% of Bisabolol (and) Farnesol, preferably 0,4 to 0,6 wt%.

6. The composition according to any one of claims 1 to 5,
wherein the composition comprises 5,0 to 5,8 wt% of Calendula Oil.

7. The composition according to any one of claims 1 to 6,
wherein the composition comprises 2,5 to 3,5 wt% of Camelina Sativa Seed Oil.

8. The composition according to any one of claims 1 to 7,
wherein the composition comprises cosmetically acceptable auxiliary substances which are selected from the group consisting of emollients, skin conditioning agents, viscosity regulating substances, fragrances, and mixtures thereof.

9. A composition for use in soothing the skin by applying it on skin areas irritated by environmental conditions
wherein the composition comprises
0,1 to 3,0 wt% of a lipophilic complex containing 0,1 to 1,0 wt% of Rosa centifolia flower extract obtainable by cryoextraction of Rosa centifolia flowers, 0,1 to 1,0 wt% of Bisabolol (and) Farnesol, 0,01 to 0,1 wt% of antioxidants, 5,0 to 6,0 wt% of Calendula Oil, 2,0 to 4,0 wt% Camelina Sativa Seed Oil,
and cosmetically acceptable auxiliary substances up to 100 wt%, whereby these auxiliary substances comprise 55 to 74 wt% of a silicone elastomer,
wherein all given percentages relate to the total weight of the composition.

10. The composition according to claim 9,
wherein the composition is applied 2 to 3 times daily.
